Europäisches Patentamt

European Patent Office

Office européen des brevets

(11) Publication number: **0 254 418**
**A2**

# (12) EUROPEAN PATENT APPLICATION

(21) Application number: 87305448.0

(22) Date of filing: 19.06.87

(51) Int. Cl.⁴: **C08J 3/08 , D01F 6/54 , D01F 8/08 , A61K 6/08**

(30) Priority: 20.06.86 GB 8615103
13.11.86 GB 8627142

(43) Date of publication of application:
**27.01.88 Bulletin 88/04**

(84) Designated Contracting States:
**BE CH DE ES FR GB IT LI NL SE**

(71) Applicant: **COURTAULDS PLC**
**18, Hanover Square**
**London W1A 2BB(GB)**

(72) Inventor: **Akers, Paul John**
**542 Binley Road**
**Binley Coventry(GB)**
Inventor: **Randall, Peter David**
**35 Chetwynd Drive**
**Whitestone Nuneaton(GB)**
Inventor: **Chambers, Pauline Barbara**
**30 Woodridge Avenue**
**Allesley Green Coventry(GB)**
Inventor: **Bell, Graeme Alan**
**18 Thorney Road**
**Wyken Coventry(GB)**
Inventor: **Wilson, Gary John**
**16 Knight Avenue**
**Coventry CV1 2AY(GB)**
Inventor: **Satgurunathan, Rajasingham**
**7 Kent Hurst Close**
**Eastern Green Coventry(GB)**

(74) Representative: **Hale, Stephen Geoffrey et al**
**J.Y. & G.W. Johnson Furnival House 14/18**
**High Holborn**
**London WC1V 6DE(GB)**

(54) **Preparation of polymer dispersions.**

(57) A polymer dispersion is prepared by dispersing polymer particles in a solvent. The polymer particles comprise one or more core particles (preferably of particle size below 0.5 micron) of a first polymer, which is insoluble in the solvent, encapsulated within a second polymer which is soluble in the solvent.

The polymer particles are generally prepared by polymerising the monomers forming the second polymer in the presence of an emulsion of the first polymer. The first polymer is preferably prepared by an emulsion polymerisation.

The dispersions are useful in fibres, films and coatings. In one embodiment the solvent is a polymerisable monomer such as methyl methacrylate forming a polymerisable moulding composition useful for denture plates, for example.

## Preparation of Polymer Dispersions

This invention relates to the preparation of polymer dispersions, for example solvent-based dispersions suitable for forming fibres, films, coatings or moulding compositions.

Many types of fibres, films and coatings are most conveniently prepared from polymer solutions. Difficulties may however be encountered in that certain monomers, which confer desirable properties on the polymer produced, give rise to polymers which have very limited solubility in common solvents. Polymers containing a high proportion of vinylidene chloride, for example, are useful in forming flame-retardant fibres and films and coatings having gas barrier properties, but they have low solubility in most solvents. The present invention prepares polymer dispersions containing a polymer which is insoluble in the disperse phase but which is of a low enough particle size to allow fibre and film formation from the dispersion.

U.S. Patent 4,202,924 describes a polymer dispersion comprising an acrylic solution polymer into which a compatible acrylic polymer, prepared for example by emulsion or suspension polymerisation, is dispersed.

A process according to the invention for the preparation of a polymer dispersion is characterised in that into a solvent are dispersed polymer particles which comprise one or more core particles of a first polymer, which is insoluble in the solvent, encapsulated within a second polymer which is soluble in the solvent.

The polymer particles are generally prepared by polymerising the monomer(s) forming the second polymer in the presence of an emulsion of the first polymer. The first polymer is preferably prepared by emulsion polymerisation. The monomer(s) for the second polymer should preferably be added without fresh surfactant so that polymerisation takes place around the particles of the first polymer.

The polymer particles may be in the form of core/shell polymer particles in which a shell of the second polymer surrounds a single core of the first polymer; the particles are generally of this type if the second polymer undergoes emulsion polymerisation and the particles remain in emulsion form throughout the polymerisation to form the second polymer. In some cases, particularly when most or all of the monomer(s) forming the second polymer are soluble to some extent in the polymerisation medium but the second polymer is not, larger polymer particles may be precipitated during polymerisation to form the second polymer. These particles generally comprise one or more (usually more) core polymer particles encapsulated in a matrix of the second polymer.

The composition of the first polymer is generally chosen to impart a desired property such as flame retardance, impact resistance or low oxygen, carbon dioxide, or water vapour permeability to fibres, films or coatings formed from the polymer dispersion. In one preferred aspect of the invention the first polymer comprises at least 70 per cent by weight, preferably at least 85 per cent by weight, vinylidene chloride to impart flame retardance. Alternatively, the first polymer may comprise at least 70 per cent by weight of an alkyl acrylate having 3 to 6 carbon atoms in the alkyl group, for example butyl acrylate, to form rubbery core particles for an impact-resistant film, coating or moulding composition. The first polymer may be cross-linked, for example it may contain 0.2 to 10 per cent by weight of units of a cross-linking monomer having 2 or more unsaturated sites such as ethylene glycol diacrylate or dimethacrylate or 1,4-butanediol dimethacrylate.

The first polymer is generally formed by conventional emulsion polymerisation using an emulsifying surfactant and a free radical initiator. The surfactant used in preparing the core polymer is preferably an anionic surfactant such as a sulphate, sulphonate, phosphate or phosphonate of a ethoxylated alkyl phenol, or it may be a non-ionic emulsifier containing one or more polyalkylene oxide chains. The free radical initiator used is preferably soluble in the aqueous phase, for example a persalt such as potassium persulphate or ammonium persulphate. The initiator can be a redox couple, such as a persulphate used with metabisulphite or dithionite. Some or all of the monomer(s) used to prepare the core polymer can be added continuously during the polymerisation, as can some or all of the initiator. A chain transfer agent such as a tertiary alkyl mercaptan may be present during polymerisation to control molecular weight. The particle size of the emulsion formed is usually below 1 micron, preferably below 0.5 micron, for example 50 to 200 nanometers (nm).

Alternatively the first polymer can be a siloxane polymer, for example a silicone elastomer. Silicone elastomers based on a cross-linked vinyl functional polyorganosiloxane are commercially available as latices with particle size below 200 nm.

The core particles may comprise particles of a first polymer as described above and also particles of a block copolymer having at least one segment compatible with the first polymer and at least one segment compatible with the second polymer. The block copolymer can for example be a block copolymer of the main monomer constituent of the first polymer and the main monomer constituent of the second polymer.

The block copolymer is preferably prepared by emulsion polymerisation. The block copolymer is preferably used at up to 10 per cent by weight, for example 1 to 5 per cent, of the total core particles. The block copolymer emulsion can be added to the emulsion of the first polymer before polymerisation of the monomer(s) forming the second polymer. The incorporation of block copolymer increases the stability of the dispersions produced according to the invention, particularly against shear which is encountered in fibre formation.

When the emulsion of the first polymer has been obtained the monomer or monomers for the second polymerisation are added and polymerisation is recommenced. The monomer(s) for the second polymerisation may be all present at the start of the second polymerisation or part can be added continbuously during polymerisation. In the latter case the proportions of monomers added during polymerisation may be different from that present at the start of polymerisation. The monomer(s) for the second polymer may be chosen from a wide range of polymerisable unsaturated monomers, for example from acrylic esters such as ethyl acrylate, butyl acrylate, methyl acrylate, propyl acrylate, hexyl acrylate, 2-ethylhexyl acrylate, hydroxy ethyl acrylate and the corresponding methacrylates, styrene, acrylonitrile, methacrylonitrile, vinyl chloride, vinylidene chloride and vinyl acetate.

In some cases the second polymer is a copolymer containing a proportion of the monomer which is the main monomer in the first polymer. For example when the first polymer is a vinylidene chloride polymer the second polymer may be a copolymer of vinylidene chloride with a comonomer such as acrylonitrile or styrene. The proportion of vinylidene chloride in the second polymer is chosen so that the second polymer is soluble in the solvent used for preparing the polymer dispersion; in some cases it may be preferred to use a proportion of vinylidene chloride which is near the maximum which allows solubility in the solvent. This can achieve a dispersion containing a higher vinylidene chloride content in the total polymer than any vinylidene chloride copolymer soluble in the solvent. Such a dispersion can form films and coatings maximising the properties such as flame retardance and gas barrier properties associated with high vinylidene chloride content.

An initiator for the second polymerisation is generally added with the monomers. In some cases free radicals may be trapped within the core particles and some polymerisation may take place on addition of the monomer or monomers for the second polymerisation even without addition of initiator. Subsequent addition of initiator is preferred in this case. When part of the monomer(s) for the second polymerisation is added continuously during polymerisation, initiator addition may continue after addition of monomer(s) to ensure a high conversion of monomer(s) to polymer. The amount of initiator used is preferably 0.05 to 1.0 per cent by weight based on the monomer(s) used for the second polymerisation. The initiator can for example be an azo compound such as azobisisobutyronitrile, a peroxide, persalt or hydroperoxide or a redox couple such as a persulphate with a metabisulphite or dithionite. A catalyst for the initiator, for example a cupric ferrous couple such as copper sulphate and ferrous ammonium sulphate used as a catalyst for a redox initiator, may be used to allow polymerisation at low temperature. The temperature of polymerisation is generally in the range 25-100°C.

The second polymer may be non-cross-linked or lightly cross-linked, for example it may contain 0.2 to 5 per cent by weight of a cross-linking monomer. If both the first and second polymers are heavily cross-linked, the polymer particles may be in the form of an interpenetrating polymer network which itself is insoluble in most solvents. If the first polymer is cross-linked but the second is only lightly cross-linked the polymer particles may be in the form of a semi-interpenetrating polymer network in which there is some entanglement between the polymer chains of the second polymer and the polymer network of the first polymer. When such a semi-interpenetrating polymer network is dispersed in solvent for the second polymer most of the second polymer dissolves in the solvent but some may remain as a solvated shell around the particles of the first polymer. This may increase the stability of the polymer dispersion in further processing.

The monomer composition used to prepare the polymeric shell can be added to a pre-formed emulsion of the core polymer or can be added during the later stages of polymerisation of the core polymer (shot growth polymerisation). In the latter process addition of monomer(s) for the second polymerisation can commence when the monomers forming the core polymer are at least 85 per cent by weight polymerised, for example about 95 per cent polymerised. Initiator is preferably added with or just before the monomer(s) for the second polymerisation. Shot growth polymerisation leads to some grafting of the second stage monomer(s) onto the core polymer. This can lead to a dispersion having a solvated shell of the second stage polymer around the core particles. Shot growth polymerisation also allows the formation of core/shell polymer with a lower overall reaction time.

The polymer particles are preferably isolated from the polymerisation medium before they are dispersed in the solvent for the second polymer. The polymer can be isolated from the emulsion by freezing and thawing or by coagulation by electrolyte, for example sodium chloride or potassium sulphate. Alternatively the core/shell polymer emulsion can be spray dried. Alternatively core/shell polymer emulsion can be dispersed in a solvent for the second polymer and the continuous phase of the emulsion (generally water) removed, but this is not preferred. Examples of solvents are acetone and dimethylformamide, which are useful for fibre and film formation and do not dissolve polymers having a high vinylidene chloride content, and hydrocarbons such as white spirit or xylene and ketones such as methylisobutyl ketone, which are widely used in coating compositions. The dispersed particles are generally of size 1 micron or less, for example 80 nm to 1 micron, depending on the degree of swelling of the core polymer and any associated semi-interpenetrating polymer networks by the solvent.

One example of a second polymer is a fibre-forming acrylonitrile polymer. Such a polymer comprises at least 50 per cent by weight acrylonitrile, preferably at least 85 per cent. The second polymer can for example be a copolymer of 86-95 per cent by weight acrylonitrile, 4-12 per cent by weight methyl acrylate and up to 3 per cent by weight of a monomer containing carboxylic or sulphonic acid groups to confer dyeability. The first polymer used with such a fibre-forming acrylonitrile polymer can be a vinylidene chloride polymer to improve flame retardance, an elastomer such as butadiene - acrylonitrile copolymer or cross-linked butyl acrylate polymer to increase fibre flexibility or a siloxane polymer to improve dirt resistance. The proportion of the first polymer forming core particles to such a fibre-forming acrylonitrile polymer can for example be 10:90 to 50:50, preferably 10:90 to 30:70 by weight. Polymer particles containing 2o to 30 per cent by weight core particles of vinylidene chloride polymer, for example, can be dispersed in a solvent for the acrylonitrile polymer to form a dispersion which has good fibre-spinning properties and substantially increased flame retardance.

When such a fibre-forming acrylonitrile polymer is used as the second polymer, it is generally precipitated as a slurry of polymer particles, for example of particle size 10-100 microns, each containing a plurality of core particles encapsulated in a matrix of the acrylonitrile polymer.

The invention includes a fibre-forming acrylonitrile polymer composition which is a dispersion comprising particles of a first polymer dispersed in a solution of the fibre-forming acrylonitrile polymer in a solvent, the particles of the first polymer being insoluble in the solvent. Examples of suitable solvents include dimethyl formamide, from which fibres can be dry spun, dimethyl sulphoxide, dimethyl acetamide, concentrated nitric acid and aqueous solution containing 40 to 60 per cent by weight preferably about 50 per cent by weight, sodium thiocyanate, or zinc chloride. The dispersions in aqueous sodium thiocyanate can be wet spun into a more dilute sodium thiocyanate solution, containing for example 8 to 20 per cent by weight sodium thiocyanate, as is well known in acrylic fibre production. Dispersions in dimethyl sulphoxide, dimethyl acetamide or nitric acid can be wet spun into aqueous solutions of the solvent. Dispersions in aqueous zinc chloride can be wet spun into more dilute zinc chloride solutions.

In another embodiment of the invention the first polymer is a silicone elastomer and the second polymer is a film-forming acrylic polymer, preferably an alkyl acrylate and/or methacrylate polymer. A dispersion of particles formed of these polymers is useful as an anti-fouling paint. Silicone elastomers are known as useful in inhibiting the adhesion of marine organisms such as algae or barnacles to an underwater surface. They have however not been widely used, particularly on ships' hulls, because they are easily mechanically damaged. The acrylic polymer used as a second polymer with such a silicone elastomer is preferably a copolymer of methyl methacrylate with an alkyl acrylate containing 3 to 6 carbon atoms in the alkyl group, for example butyl acrylate, preferably in the weight ratio 50:50 to 80:20. Such copolymers can form tough films. When particles of silicone elastomer encapsulated in the acrylate and/or methacrylate ester in the polymer are dispersed in a solvent such as methylisobutyl ketone, the acrylate polymer dissolves in the solvent whilst the silicone elastomer is not dissolved. When the dispersion is cast as a film or coated on a surface the acrylate polymer initially forms the continuous phase but there is phase separation of the polymers. The low energy silicone polymer moves to the free surface of the film, so that the film or coating formed gains mechanical strength from the acrylate polymer but has a low energy surface of the silicone which inhibits marine fouling. The silicone elastomer is preferably based on a cross-linked vinyl functional polyorganosiloxane which has residual vinyl functionality. This allows some grafting of the acrylate or methacrylate monomers on the polysiloxane, aiding the retention of the polysiloxane at the surface of the film.

In another embodiment of the invention, particularly useful for making moulding compositions, the solvent in which the polymer particles are dispersed is a polymerisable monomer. Examples of suitable monomers are methacrylate and acrylate monomers such as methyl methacrylate, butyl methacrylate and ethyl acrylate, and styrene. The dispersion formed comprises core particles of the first polymer dispersed in a solution of the second polymer in the polymerisable monomer. This composition can be shaped, for example by injection, extrusion or compression moulding, and heated to polymerise the monomer acting as solvent. The moulded product comprises core particles of the first polymer dispersed in a matrix which is a blend of the second polymer and a polymer of the monomer acting as a solvent. The core particles can, for example, be rubbery particles which increase the impaact strength of the moulded product. The moulding compositions are particularly suitable for producing denture plates.

The material traditionally used for forming acrylic dentures is a moulding composition formed from an acrylic polymer powder, generally a methyl methacrylate polymer, and methyl methacrylate monomer. The methyl methacrylate monomer is polymerised as the composition is moulded. The denture plates produced have the disadvantage that the methyl methacrylate polymer used has poor impact strength. Dentures formed from it break easily if dropped or may even break during mastication of hard foods. There have been suggestions to improve the impact strength of denture plates by incorporation of a butadiene-based rubbery material, for example as described in US Patent 3427274, or by micro-dispersion of a rubber phase. Such modified denture forming compositions have however been found difficult to mould into uniform denture plates.

A moulding composition according to one embodiment of the invention for use in the preparation of denture plates comprises a mixture formed from a polymer powder and methyl methacrylate monomer and is characterised in that the polymer powder consists of composite particles having a shell of a hard acrylic polymer composed of at least 60% by weight polymerised methyl methacrylate surrounding a core comprising a rubbery acrylic polymer which is a cross-linked polymer of an alkyl acrylate having 3 to 6 carbon atoms in the alkyl group. The polymer particles are preferably produced by core/shell latex polymerisation.

The rubbery acrylic polymer which forms the core of the composite polymer particles is preferably composed of at least 70% by weight of the $C_3-C_6$ alkyl acrylate and 0.5 to 10% by weight of units of a cross-linking monomer having 2 or more unsaturated groups, such as ethylene glycol diacrylate or dimethacrylate or 1,4-butane-diol dimethacrylate. Most preferably the rubbery acrylic poolymer comprises 92 to 98% butyl acrylate and 2 to 8% of the cross-linking monomer, by weight.

The monomer composition used to prepare the shell preferably comprises 60 to 90% by weight of methyl methacrylate with 10 to 40% by weight of another acrylic monomer such as an alkyl acrylate or methacrylate. The monomer composition more preferably comprises 70 to 85% by weight methyl methacrylate with 15 to 30% by weight of an alkyl acrylate having 3 to 6 carbon atoms in the alkyl group, for example butyl acrylate. The monomer composition preferably includes a small proportion, for example 0.2 to 5% by weight, of a cross-linking monomer such as a diacrylate or dimethacrylate. When such a cross-linking monomer is used in the shell polymer as well as in the core polymer the polymer particles may be partly in the form of a latex-interpenetrating network. The degree of cross-linking should however be low enough that most of the shell polymer dissolves in methyl methacrylate monomer. Some of the hard acrylic polymer may remain as a solvated shell around the core particles when the polymer powder is dispersed in methyl methacrylate; this may give a more consistent improvement in impact strength. The monomers forming the shell polymer are preferably such as to form a polymer having a glass transition temperature Tg in the range 50 to 100°C, preferably 60 to 85°C.

The proportion of core polymer to shell polymer is preferably in the range 10:90 to 50:50 by weight, most preferably 20:80 to 40:60. The hardness of the core/shell polymer particles formed is preferably such that if moulded separately they have a Shore D hardness of 60 to 85, most preferably 70 to 80. The particle size of the core/shell polymer is preferably in the range 0.1 to 0.5 microns.

The glass transition temperature Tg of the core/shell polymer, as measured by differential scanning calorimetry on a small sample of the polymer in fine powder form, is preferably in the range of 50 to 100°C, most preferably 60 to 85°C.

The core/shell polymer particles may be formed by a 3-stage polymerisation process. In this case the polymer emulsion formed by the first two stages of polymerisation is to be regarded as the core polymer, that is to say this polymer should include a rubbery acrylic polymer which is a cross-linked polymer of a $C_3$-$C_6$ alkyl acrylate and the shell polymer formed by the third stage of polymerisation should be a hard acrylic polymer comprising at least 60% by weight polymerised methyl methacrylate. The polymer particles can for example comprise an inner core of a cross-linked methyl methacrylate/butyl acrylate polymer containing 50 to 85% methyl methacrylate and 15 to 50% butyl acrylate, by weight, with a second layer forming the outer part of the core comprising cross-linked poly(butyl acrylate). The second stage of polymerisation is preferably carried out without surfactant so that the core polymer is generally a latex-interpenetrating

5

polymer network. The weight ratio of the inner core polymer to the cross-linked butyl acrylate layer forming the outer portion of the core is preferably 15:85 to 60:40 by weight The shell polymer is preferably a methyl methacrylate copolymer formed as described above. The weight ratio of the total core polymer to the shell polymer is preferably 20:80 to 50:50.

The moulding composition of this embodiment of the invention is generally prepared by mixing the core-shell polymer powder and methyl methacrylate monomer to form a mouldable gel. The preferred ratio of mixing is between 1:2 and 4:1 by weight polymer powder to monomer. Mixing can for example be carried out by an 'Ivoclar Cap-vibrator' mechanical mixing device. The optimum weight ratio of polymer powder to monomer may be in the range of 1:1 to 3:1 or in the range of 1:2 to 1:1 depending on the moulding technique to be used. The moulding composition generally contains a free radical initiator for the polymerisation of the methyl methacrylate monomer, for example an organic peroxide such as benzoyl peroxide or lauroyl peroxide. The amount of initiator is preferably 0.2 to 2 per cent by weight based on the amount of monomer in the composition, for example about 0.5 per cent.

The method of making acrylic dentures by the lost wax process in a denture flask is well known to dental practitioners and technicians. A mouth impression is first reproduced in dental stone mould material and artificial teeth are set in wax to form a replica denture. After trial fitment of this wax denture in the mouth of the patient, it is then sealed with wax to the stone model cast whose base is set in a gypsum investment with the teeth protruding from half of the denture flask. When the investment has hardened the surfaces are coated with a separating medium. The remaining half of the denture flask is filled with more gypsum investment and assembled with the first part containing the wax replica denture for moulding. The complete denture flask is next immersed in boiling water for a short period, the two halves separated and the wax eliminated with boiling water and detergent. All surfaces, apart from the teeth, are coated with a mould sealer prior to moulding the acrylic resin.

Moulding has traditionally been carried out by compression moulding. The compression moulding technique has disadvantages; in particular shrinkage of the composition as the methyl methacrylate polymerises produces a denture which does not conform accurately to the mould and may be a poor fit in the mouth. Despite its disadvantages, compression moulding has remained in wide spread use because of its simplicity.

Pressure injection moulding methods for producing acrylic dentures have been described in US Patent 2806253, British Patent 1442041 and UK Patent Application 2159457. These processes are capable of producing more precise denture mouldings. They generally include means for injecting further moulding compositions to compensate for shrinkage during polymerisation.

The present invention includes a process in which a moulding composition according to the embodiment of the invention defined above is injected into a mould for a denture plate having means for injecting further moulding composition during moulding and polymerisation, and is heated to polymerise the methyl methacrylate and to mould the composition to form a moulded polymerised product, at least part of the heating being at a temperature above the glass transition temperature of the moulded polymerised product.

The moulding composition of the invention can be used to prepare denture plates by compression moulding but is particularly suitable for the preparation of denture plates by injection moulding. It is especially suitable for injection moulding using the apparatus described in UK Patent Application 2159457. In this apparatus the gypsum mould produced by the lost wax process is located in a chamber defined between a pair of plates spaced apart by a resilient body. The neck of the resilient body carries an injection cylinder whose nozzle extends into the interior of the mould container so that the injection of the polymerisable moulding composition into the mould can continue as the composition shrinks during polymerisation.

In the process described in UK Patent Application 2159457 the moulding composition is injected into the mould space and the temperature is raised to a temperature sufficient to effect controlled polymerisation accompanied by material flow, for example a temperature of 60 to 70°C. Volume reduction of the aterial, compensated by injection of further moulding composition as polymerisation proceeds, occurs as polymerisation takes place. When this is substantially complete, for example after 1.5 to 2.5 hours, the temperature is raised, for example to 90 to 120°C, to complete the polymerisation process.

This two-stage polymerisation of the moulding composition can be used when moulding and polymerising the composition of the present invention, but it is not necessary provided that the moulding is carried out at a temperature above the Tg of the denture plate polymer formed. The denture plate polymer has a higher Tg than the core/shell polymer powder because the polymerisation of the methyl methacrylate monomer in the moulding composition leads to a hard polymer. The denture plate polymer absorbs energy over a wide range of temperature above about 70°C, as is characteristic of impact-resistant polymers, and has a Tg generally in the range of 75-105°C, preferably 85-100°C. Moulding preferably takes place at 90-130°C, most preferably 105-120°C. Heating is preferably carried out for 0.5 to 3 hours, most preferably 1 to 2 hours. The moulded denture plate is cooled in the mould to a temperature below its Tg; forced draught cooling, for example by a fan, may be used.

The polymerisable moulding composition of the invention can also be used in the process and apparatus described in British Patent 1442041, in which the polymerisable composition is injected into a mould cavity and polymerised at a rate which decreases towards the injection aperture. Additional polymerisable composition is injected into the cavity during polymerisation to compensate for shrinkage. The gradient of the rate of the polymerisation is achieved by establishing a temperature gradient, for example by insulating the souce of heat from the surface of the mould near the injection aperture and from the reservoir of polymerisable composition. The proportion of polymer powder to methyl methacrylate monomer in the moulding composition is at the lower end of the preferred range when using this process, for example 1:2 to 1:1 by weight.

The moulded denture preferably has a Shore D hardness of 70 to 85, more preferably 75 to 80. Moulded dentures can be produced according to the invention having a Tg and a hardness within this desired range combined with a Hounsfield notched impact resistance measurement in the range of 0.07 to 0.15 or even higher. This compares with a Hounsfield notched impact resistance measurement of 0.025 to 0.035 for conventional moulded dentures based on polymerisable moulding compositions formed of methyl methacrylate polymers and methyl methacrylate monomer.

The invention is illustrated by the following examples in which parts and percentages are by weight.

## EXAMPLE 1

### (i) Production of First Polymer

12 g of Ethylan BGH 86 (trade mark), a non-ionic polyethoxylate emulsifier, and 4 g of sodium methallyl sulphonate (SMS) were dissolved in 700 g of deoxygenated water. 2 ml of copper sulphate solution (25 g/l) and 0.8 ml of ferrous ammonium sulphate solution (39.2 g/l) were added as catalysts for the initiator redox couple. The resulting mixture was stirred in a 3 litre reaction vessel fitted with a stirrer (paddle type), cold trap, nitrogen inlet, and initiator feed and monomer feed inlets.

120 g of vinylidene chloride (VDC), 16 g of acrylonitrile (AN) and 7.5 g of ethyleneglycol dimethacrylate (EGDM) were added to this vessel and the mixture stirred for 1 hour under a nitrogen atmosphere. The pH of the mixture was adjusted to 3.65 using dilute sulphuric acid.

2 ml each of ammonium persulphate (AP) (12.5 g/250 ml) and sodium metabisulphite (SM) (5.4 g/250 ml) solutions were added as initiators and feeds of 3 ml per 10 minutes of each of these initiators were started. Initiation occurred after 10 minutes and the monomer feed (totalling 60 g VDC, 8 g AN, 3.7 g EGDM) was started. Monomers were fed at a rate of 10 ml/10 minutes, over 80 minutes, and the initiators over 110 minutes. The latex was stirred for a further 30 minutes. The particle size of the latex was 136 nm.

### (ii) Production of Second Polymer

The second stage bulk monomers (119 g VDC, 178 g AN, 27 g SMS) were added to the above latex along with an extra 100 g of water. The resulting mixture was stirred for a further 60 minutes during which time a rapid and large exotherm was observed.

2 ml each of AP and SM solutions (at the same concentrations as above) were added as initiators and the feed of these initiators started. As soon as initiation occurred a monomer feed (totalling VDC 182 g, AN 162 g) was started. These monomers were fed at a rate of 15 ml/10 minutes, over 230 minutes, and the initiators at a rate of 3ml/10 minutes, over 330 minutes. In adddition, 180 ml of water was added in 10 ml aliquots every 10 minutes. The reaction mixture was observed to slurry 100 minutes after the addition of the bulk monomers. The slurry was stirred for a further 30 minutes after the initiator feed stopped. The reaction was then terminated by the addition of 11 ml Nervanaid (trade mark) solution (2.5 g/250 ml).

The slurry was filtered through a 10 micron cloth, then dried in an oven at 50°C. No latex was recovered in the filtrate.

(iii) Preparation of Dispersions

Samples of the dried polymer particles, having a content of polymerised VDC units of 54 per cent by weight, were dispersed in acetone and in dimethyl formamide at 25 per cent by weight. Each solvent dissolved the shell polymer, but left the core polymer finely dispersed throughout the solution. The average particle size of the dispersed particles in acetone was 0.4 micron. The dispersions could be cast to give hard, colourless, slightly opaque films, whereas ordinary VDC/AN copolymers containing 54 per cent by weight VDC units are insoluble in acetone.

These films show improved flame retardancy compared with films formed from a solution of the VDC/AN copolymer used as the shell polymer as measured by the Limited Oxygen Index (LOI) of the cast films. Films produced from the dispersion according to the invention had a LOI of 31.3 compared with 27.7 for films of the VDC/AN copolymer.

Example 2

(i) Production of First Polymer

29.1 g of an anionic emulsifier were dissolved in 650 g of deoxygenated water and 50 g of isopropanol. 4.1 ml copper sulphate solution (25g/litre) and 0.8 ml ferrous ammonium sulphate solution (39.2g/litre) were added. The resulting solution was stirred in a 3 litre reaction vessel as described in Example 1.

90.2g VDC, 2.0 g AN and 3.84 g EGDM were then added to the vessel and the mixture stirred for 10 minutes under nitrogen. The pH of the mixture was then adjusted to 3.65 using dilute sulphuric acid.

4 ml each of AP (6.25 g/250 ml) and SM (2.7 g/250 ml) solutions were added as initiator and initiator feeds of 3.12 ml/10 mins of each of the initiator solutions started. As soon as initiation took place the monomer feed (360.8 g VDC, 8.0 g AN and 15.36 g EGDM) was started. Monomers were fed at a rate of 26.7 ml/10 mins over 2 hours and the initiators over 2.5 hours. The latex was stirred for a further 30 minutes. The particle size was 78 nm.

(ii) Production of Second Polymer

The second stage bulk monomers (88.0 g VDC, 64 g styrene and 6.0 g AN) were added to the above latex. The mixture was stirred for 30 minutes.

4ml each of the AP and SM initiator solutions used in stage (i) above were added. Initiator feeds of 8.89 ml/10 mins each were then started. As soon as initiation took place the monomer feed (88.0g VDC, 64.0g styrene and 6.0g AN) was started. Monomers were fed at a rate of 10.7mls/10 mins over 2.5 hours and the initiators over 3 hours. The latex was stirred for a further 15 minutes then polymerisation was terminated by the addition of 'Nervanaid' solution.

The latex, which comprised core/shell polymer particles, was filtered through a 10 micron cloth. The solid polymer was recovered by adding an equal volume of sodium chloride solution (1.5 M) to the latex. The slurry produced was filtered and the polymer washed with water. The polymer was dried in a vacuum oven at 45-50°C.

(ii) Preparation of Dispersion

The polymer was dispersed at 25 per cent by weight in xylene/methyl ethyl ketone (1:1 by volume) which dissolved the shell copolymer of styrene and VDC but left the polyvinylidene chloride core finely dispersed throughout the solution. The average size of the dispersed particles was 90 nm. The dispersion formed smooth surface coatings on many substrates. The overall VDC content of the polymers in the dispersion was 80 per cent by weight; copolymers of VDC and styrene are not soluble in xylene/ketone solvents at a VDC content above 65 per cent.

8

Example 3

(i) Production of First Polymer

24.6 g of an anionic emulsifier were dissolved in 850 g of deoxygenated water. 6.5 ml copper sulphate solution (25g/l) and 1.3 ml ferrous ammonium sulphate solution (39.2g/l) were added. The resulting solution was stirred in a 3 litre reaction vessel as described in Example 1.

240g butyl acrylate and 9.6 g EGDM were added to the vessel and the mixture stirred for 10 minutes under nitrogen. The pH of the mixture was adjusted to 3.65 using dilute sulphuric acid.

4ml each of AP (6.25g/250 cm³) and SM (2.7g/250 cm³) initiator solutions were added and initiator feeds of 4ml/10 mins each started. As soon as initiation took place the monomer feed (240 g butyl acrylate, 9.6 EGDM) was started. Monomers were fed at a rate of 22.9 mls/10 mins over 2 hours and the initiators over 2.5 hours.

The latex was then stirred for a further 30 minutes. The particle size was 69nm.

(ii) Production of Second Polymer

The second stage bulk monomer (160 g methyl methacrylate) was added to the above latex. The mixture was stirred for 30 minutes.

4ml each of the AP and SM initiator solutions were added. Initiator feeds of 4ml/10 mins each were then started. As soon as initiation took place the monomer feed (160 g methyl methacrylate) was started. Monomer was fed in at a rate of 9.4 ml/10 mins over 3 hours and the initiators over 3.5 hours. The latex was then stirred for 15 minutes then 'Nervanaid' solution was added. The latex, which comprised core/shell polymer particles, was filtered through a 10 micron cloth. The solid polymer was recovered by adding an equal volume of sodium chloride solution (1.5 M) to the latex. The slurry produced was filtered and the polymer washed with water. The polymer was dried in a vacuum oven at 45-50°C.

(iii) Preparation of Dispersion

The polymer was dispersed at 25 per cent by weight in methyl ethyl ketone which dissolved the polymethylmethacrylate shell but left the polybutyl acrylate core finely dispersed throughout the solution. The average size of the dispersed particles was 1.0 micron. The dispersion formed smooth impact-resistant surface coatings on many substrates.

Example 4

(i) Preparation of Second Polymer

In this example the core latex used was a commercially available latex, with a high VDC comonomer content (greater than 90 per cent). It has a particle size of 78 nm and a solids content of 50 per cent by weight.

160 g latex was mixed with 1420 g of deoxygenated water. 1.0g of ammonium sulphate in 100 g water was added. 2 ml of copper sulphate solution (25g/l) and 0.8 ml ferrous-ammonium sulphate solution (39.2g/l) were added. The resulting mixture was stirred in a 3 litre reaction vessel, as described in Example 1.

106g AN and 10.5g of methyl acrylate (MA) were added and the mixture stirred for 1 hour under nitrogen.

5.0ml each of AP (6.7g/250 mls) and SM (2.7g/250ml) initiator solutions were added and initiator feeds of 3ml/10 minutes of each solution started. Initiation occurred after 5 minutes and the monomer feed (106 g AN, 10.5g MA) was then started at a rate of 10ml/10 minutes, over 120 minutes. Initiators were fed over 150 minutes. A slurry of polymer particles was produced, the particles each comprising core latex particles in a matrix of AN/MA copolymer. The ammonium sulphate added to the latex stabilises the slurry so that larger polymer particles are formed. The slurry produced was stirred for 120 minutes. The slurry was filtered through a 10 micron cloth and no latex was found in the filtrate.

9

(ii) Preparation of Dispersion

Samples of the polymer particles were dispersed at 13 per cent by weight in 52 per cent by weight aqueous sodium thiocyanate and in dimethyl formamide. Both solvents dissolved the matrix polymer but left the core particles finely dispersed throughout the solution. The particle size of the dispersed core polymer in sodium thiocyanate solution was 158 nm. The overall VDC content of the polymers in the dispersion was 25 per cent by weight; VDC/AN copolymers containing more than 10 per cent VDC are not soluble in sodium thiocyanate solution.

The dispersion in dimethyl formamide was cast to give white films. These films exhibited significant improvements in flame retardance, with respect to the AN/MA shell polymer, as measured by the Limiting Oxygen Index (LOI) of the cast films. Films formed from the dispersion produced according to the invention had a LOI of 21.3 compared to 18.4 for the AN/MA polymer.

The dispersion in sodium thiocyanate solution was formed into 4 decitex fibres by wet spinning into dilute (12 per cent by weight) aqueous sodium thiocyanate. The fibres formed had a tenacity of 16.9 CN/tex and a LOI of 23.9, compared to 20.7 for fibres formed from the AN/MA shell polymer.


Example 5

In this example the core latex used was a commercially available butadiene-acrylonitrile copolymer (32 per cent by weight acrylonitrile) latex, with a solids content of 40 per cent.

40g latex was mixed with 1500g of deoxygenated water. The resulting mixture was stirred in a 3 litre reaction vessel fitted with stirrer, cold trap and nitrogen inlet. 212g AN and 21g MA were added and the mixture stirred for 1 hour under a nitrogen atmosphere. During this time the pH of the mixture was adjusted to 7.5, and the temperature raised to 80°C. 1.5g potassium persulphate was added. Initiation occurred after 10 minutes, the reaction mixture slurrying after 30 minutes. The mixture was left for a total of 360 minutes to react, after which time it was cooled and filtered through a 10 micron cloth. No latex was observed in the filtrate.

Samples of the polymer were dispersed at 12 per cent by weight in 52 per cent by weight aqueous sodium thiocyanate. The solvent dissolved the shell polymer but left the core finely dispersed throughout the solution. The overall core content was 5 per cent of the total polymer weight.

The dispersion could be extruded to form fibres of increased flexibility.


EXAMPLE 6

In this example the core latex used was a silicone elastomer latex (Dow Corning) based on a cross-linked vinyl functional polydiorganosiloxane and having a particle size of about 100 nm.

30 parts by weight (polymer solids basis) of the latex was diluted to 12 per cent solids with distilled water containing 0.5 parts by weight potassium persulphate and heated to 80°C. A monomer blend of 49 parts by weight methyl methacrylate and 21 parts butyl acrylate was added over one hour maintaining the temperature at 80°C. A latex of core/shell polymer particles was formed of average particle size 137 nm.

The polymer was isolated from the latex by freezing and thawing. A sample of the polymer was dissolved in methyl isobutyl ketone and cast to form a tough film having a low energy surface of the silicone elastomer.


Example 7

Stage 1: Preparation of Poly-butyl acrylate Core

The reaction was conducted in a 2 litre reactor flask, fitted with a cold trap, nitrogen inlet, thermocouple, stirrer (paddle type) and monomer feed inlet.

Perlankrol FN65 (4.5g) (an ethoxylated alkyl phenol sulphonate emulsifier) was dissolved in 600g of water. The pH was adjusted to 7.8 with dilute sodium hydroxide solution and the solution was stirred for 30 minutes. 2 ml of tertiary dodecyl mercaptan (chain transfer agent) was added to the solution. Potassium persulphate (1g) (initiator) was dissolved in the mixture. 10 g of a monomer blend consisting of butyl acrylate (384g) and ethylene glycol dimethacrylate (EGDM) (16 g) was added to the emulsifier/initiator solution. The temperature was raised to 85°C and maintained for 5 minutes; then the source of heat was switched off.

The remainder of the monomer blend mentioned above was added at a rate sufficient to maintain the temperature at 85°C. Monomer addition was completed in 50 minutes. The temperature was held at 85°C for 30 minutes after completion of monomer blend addition. The latex was then cooled and filtered. The particle size of the latex was 110 nm.

Stage 2: Preparation of the Shell/Core Polymer

The apparatus used was similar to stage 1. 500g of the core latex was mixed with distilled water (450g) containing dissolved potassium persulphate (2g). The mixture was stirred under nitrogen for 30 minutes. A monomer blend consisting of methyl methacrylate (240 g), butyl acrylate (60 g) and EGDM (3 g) was made up. The temperature was raised to 80°C, maintained for 5 minutes, then the source of heat switched off. The monomer blend was then added at a rate sufficient to maintain the tempertaure at 80°C. Monomer addition was completed in 1 hour. The temperature was then held at 80°C for 1 hour after all the monomer blend had been added. The latex was cooled and filtered.

The polymer was isolated from the latex by freezing and thawing . A sample moulded from the polymer powder had a Shore D hardness of 65. The Tg of the polymer powder was measured as 69°C.

The polymer powder produced was mixed with methyl methacrylate monomer in a ratio 3 parts to 1 and with 0.5 per cent benzoyl peroxide based on the methyl methacrylate monomer. The composition was then injection moulded to form dentures using the process and apparatus described in the UK patent application 2159457. Moulding was carried out at 110-115°C and a polymer face pressure of 300-350 pounds per square inch for 90 minutes, followed by controlled fan cooling in the mould for 30 minutes. The dentures produced had a Houndsfield impact resistance measurement of 0.17, a Shore D hardness of 75 and a Tg of 85°C.

Examples 8 to 12

Stage 1 of Example 7 was repeated to prepare an emulsion of cross-linked polybutyl acrylate core polymer. Samples of this emulsion were used to prepare a series of core/shell polymer emulsions having the compositions set out in Table 1 below, using the process described in Stage 2 of Example 7. Each core/shell polymer was isolated as a powder, mixed with methyl methacrylate and initiator, and moulded to form denture plates as described in Example 7. The Hounsfield impact resistance of the plates formed is given in Table 1.

## Table 1

| Example No | Weight Ratio of Core to Shell | Composition of Shell Polymer | | | Tg of Polymer Powder ($^{\circ}$C) | Houndsfield Resistance of Moulded Plate |
|---|---|---|---|---|---|---|
| | | MMA[†] | BA[†] | EGDM | | |
| 8 | 20:80 | 80 | 20 | 1 | 88 | 0.07 |
| 9 | 20:80 | 75 | 25 | 1 | 72 | 0.11 |
| 10 | 30:70 | 75 | 25 | 1 | 69 | 0.08 |
| 11 | 30:70 | 80 | 20 | 1 | 76 | 0.09 |
| 12 | 40:60 | 75 | 25 | 1 | 60 | 0.12 |

(† MMA = Methyl Methacrylate; BA = Butyl acrylate)

0 254 418

## Example 13

### (a) Preparation of Inner Core Emulsion

75 parts methyl methacrylate, 25 parts butyl acrylate and 1 part EGDM were emulsion polymerised in 150 parts water using the process described in Stage 1 of Example 7 at a temperature of 80°C. The emulsion formed had a particle size of 119 nm.

### (b) Preparation of Core Polymer Emulsion

Half of the emulsion prepared in (a) above was mixed with a solution of potassium persulphate and butyl acrylate (192 parts) and EDGM (8 parts) were copolymerised using the process of Stage 2 of Example 7 at a temperature of 85°C. The emulsion formed consisted of particles having a cross linked butyl acrylate layer surrounding a methyl methacrylate copolymer core. The particle size of the emulsion was 222 nm.

### (c) Preparation of Core/Shell Polymer

A portion (20 parts polymer) of the emulsion prepared was mixed with a solution of potassium persulphate and methyl methacrylate (60 parts), butyl acrylate (20 parts) and EGDM (0.8 parts) were copolymerised using the process of Stage 2 of Example 7. The emulsion formed consisted of core/shell polymer articles of particle size 384 nm

The polymer was isolated as a powder as described in Example 7. Its Shore D hardness was 73. The polymer powder could be mixed with methyl methacrylate monomer and initiator and injection moulded to form denture plates as described in Example 7. The denture plates formed had increased impact resistance compared to plates moulded from polymethyl methacrylate with methyl methacrylate.

## Examples 14 to 16

Polymer powders were prepared using the process of Example 13. In each case , the composition of the inner core, of the cross-linked polybutyl acrylate layer and of the shell polymer was as described in Example 13 but the ratio between the polymer layers was varied as set out in table 2. All the polymer powders could be injection moulded with methyl methacrylate to form denture plates of improved impact resistnce; the samples were ranked (best first) Examples 14 > 16 > 13 > 15 by impact resistance.

## Table 2

| Example No. | Inner Core : 1st Shell : 2nd Shell Ratio | | | Latex Paticle Size/nm | | | Shore Hardness |
|---|---|---|---|---|---|---|---|
| | Inner Core | 1st Shell | 2nd Shell | Core | 1st Shell | 2nd Shell | |
| 13 | 4 | : 16 | : 80 | 119 | 222 | 304 | 74 |
| 14 | 8 | : 32 | : 60 | 119 | 222 | 207 | 62 |
| 15 | 10 | : 10 | : 80 | 119 | 163 | 201 | 77 |
| 16 | 20 | : 20 | : 60 | 119 | 163 | 212 | 74 |

0 254 418

Example 17

The reaction was conducted in a 3 litre reactor flask fitted with a cold trap, nitrogen inlet, thermocouple, stirrer (paddle type) and monomer feed inlet.

Perlankrol FN65 emulsifier (4.5 g) was dissolved in 1150 g of deoxygenated water. The pH was adjusted to 7.8 with dilute sodium hydroxide solution and 2 ml of tertiary dodecyl mercaptan (transfer agent) was added to the solution. 50 ml of a monomer blend consisting of butylacrylate (384 g) and EGDM (16 g) was added to the emulsifier solution. The temperature was raised to 60°C and then the initiator solution (1.0 g Ammonium persulphate (AP) in 10g water) was added to the emulsified monomer mixture. The temperature was raised to 85°C, assisted by the reaction exotherm and the heat source switched off.

The remainder of the monomer blend was added at a rate sufficient to maintain the reaction temperature of 85°C. After completion of monomer blend addition in 60 minutes the temperature was held at 85°C for 15 minutes, after which time the polymerisation had attained ca. 95% completion.

A fresh initiator solution (4 g AP in 20 g water) was added and after 5 minutes the second monomer blend (totalling 480 g methyl methacrylate, 120 g butyl acrylate and 6 g of EGDM) was added at a rate sufficient to maintain the reaction temperature at 80°C. Monomer addition was completed in 60 minutes and the temperature was held at 80°C for 60 minutes to allow complete conversion.

The emulsion was cooled and filtered and the polymer was isolated as described in Example 7. The polymer was injection moulded with methyl methacrylate as described in Example 7 to give denture plates of properties similar to those of the plates of Example 7.

Example 18

An emulsion of cross-linked poly(butyl acrylate) core polymer particles was prepared as described in Example 7. Core/shell polymer particles were prepared using the process of Stage 2 of Example 7 with a second stage monomer composition comprising MMA, BA and EGDM in weight ratio 80:20:2 and a weight ratio of core polymer to shell polymer of 35:65. The particle size of the polymer powder formed was 145 nm and it had a Tg of 75°C.

The polymer was mixed with methyl methacrylate monomer in an 'Ivoclar Cap-vibrator' at a polymer to monomer weight ratio of 2:3 to form a gel. The gel was moulded into denture plates using the procedure of UK Patent Application 2159427 and using that of British Patent 1442041. In both cases impact resistant plates were produced having a Hounsfield impact resistance of 0.11 and a Tg of 100°C.

**Claims**

1. A process for the preparation of a polymer dispersion, characterised in that into a solvent are dispersed polymer particles comprising one or more core particles of a first polymer, which is insoluble in the solvent, encapsulated within a second polymer which is soluble in the solvent.

2. A process according to claim 1, characterised in that the polymer particles are core/shell polymer particles which each consist of a shell of the second polymer surrounding a single core of the first polymer.

3. A process according to claim 1, characterised in that the polymer particles comprise more than one core particle encapsulated in a matrix of the second polymer.

4. A process according to any of claims 1 to 3, characterised in that the core particles have a particle size below 0.5 micron.

5. A process according to any of claims 1 to 4, characterised in that the first polymer has been prepared by emulsion polymerisation.

6. A process according to any of claims 1 to 5, characterised in that the first polymer comprises at least 70 per cent by weight vinylidene chloride.

7. A process according to any of claims 1 to 5, characterised in that the first polymer is a rubbery polymer comprising at least 70 per cent by weight of an alkyl acrylate having 3 to 6 carbon atoms in the alkyl group.

8. A process according to any of claims 1 to 7, characterised in that the first polymer is a cross-linked polymer.

9. A process according to any of claims 1 to 8. characterised in that the core particles comprise particles of a first polymer and particles of a block copolymer having at least one segment compatible with the first polymer and at least one segment compatible with the second polymer.

10. A process according to any of claims 1 to 4, characterised in that the first polymer is a silicone elastomer.

11. A process according to claim 6, characterised in that the second polymer is a film-forming vinylidene chloride copolymer having a lower vinylidene chloride content than the first polymer.

12. A process according to any of claims 1 to 9, characterised in that the second polymer is a fibre-forming acrylonitrile polymer.

13. A fibre-forming polymer composition based on acrylonitrile polymer, characterised in that the composition is a dispersion comprising particles of a first polymer dispersed in a solution of the fibre-forming acrylonitrile polymer in a solvent, the particles of the first polymer being insoluble in the solvent.

14. A fibre-forming polymer composition according to claim 13, characterised in that the particles of the first polymer have a particle size from 80 nm to 1 micron.

15. A fibre-forming polymer composition according to claim 13 or 14, characterised in that the solvent is an aqueous solution of sodium thiocyanate.

16. A fibre-forming polymer composition according to claim 13 or 14, characterised in that the solvent is dimethyl formamide.

17. A process for forming acrylic fibres, characterised in that a fibre-forming polymer composition according to claim 15 is spun into a more dilute aqueous solution of sodium thiocyanate.

18. A process for forming acrylic fibres, characterised in that a fibre-forming polymer composition according to claim 16 is dry spun.

19. Acrylic fibres of reduced flammability, characterised in that the fibres comprise particles of a vinylidene chloride polymer dispersed in a continuous phase of an acrylonitrile polymer.

20. A process according to claim 1, characterised in that the solvent is a polymerisable monomer.

21. A process according to claim 20, characterised in that the solvent is a methacrylate ester.

22. A moulding composition for use in the preparation of denture plates comprising a mixture formed from a polymer powder and methyl methacrylate monomer, characterised in that the polymer powder consists of composite particles having a shell of a hard acrylic polymer composed of at least 60 per cent by weight polymerised methyl methacrylate surrounding a core comprising a rubbery acrylic polymer which is a cross-linked polymer of an alkyl acrylate having 3 to 6 carbon atoms in the alkyl group, the hard acrylic polymer being at least partly dissolved in the methyl methacrylate monomer and the rubbery acrylic polymer core particles being substantially undissolved in the methyl methacrylate monomer.

23. A moulding composition according to claim 22, characterised in that the hard acrylic polymer has a glass transition temperature in the range of 60 to 85°C and is a copolymer of 70 to 85 per cent methyl methacrylate with 15 to 30 per cent by weight of an alkyl acrylate having 3 to 6 carbon atoms in the alkyl group.

24. A moulding composition according to claim 22 or claim 23, characterised in that the rubbery acrylic polymer comprises at least 70 per cent by weight of butyl acrylate and 2 to 8 per cent by weight of a cross-linking monomer having 2 or more polymerisable unsaturated groups.

25. A moulding composition according to any of claims 22 to 24, characterised in that the core comprises an inner core of a cross-linked methyl methacrylate copolymer and an outer core layer of the rubbery acrylic polymer.

26. A moulding composition according to any of claims 22 to 25, characterised in that the weight ratio of core polymer to shell polymer is in the range of 10:90 to 50:50 by weight.

27. A moulding composition according to any of claims 22 to 26, characterised in that the weight ratio of polymer powder to methyl methacrylate monomer is in the range 1:1 to 4:1.

28. A moulding composition according to any of claims 22 to 26, characterised in that the weight ratio of polymer powder to methyl methacrylate monomer, is in the range 1:2 to 1:1.

29. A process for moulding a denture plate, characterised in that a moulding composition according to any of claims 22 to 28 is injected into a mould for a denture plate and is heated to polymerise the methyl methacrylate and to mould the composition to form a moulded polymerised product, at least part of the heating being at a temperature above the glass transistion temperature of the moulded polymerised product.

30. A process according to claim 29, characterised in that the mould for the denture plate has means for injecting further moulding composition and that further moulding composition according to any of claims 22 to 28 is injected during moulding and polymerisation.